# EUROPEAN PATENT APPLICATION

(11) **EP 3 278 831 A1**
(43) Date of publication of application: **07.02.2018**
(21) Application number: 17182887.4
(22) Date of filing: 25.07.2017
(51) Int. Cl.: A61M 15/00, B05B 11/00, A61M 11/08

(54) **AEROSOL SPRAY DEVICE RESEMBLING A WRITING INSTRUMENT**

(30) Priority: 05.08.2016 US 201615229946
(71) Applicant: Shlaferman, Alexander, Brooklyn, NY 11235 (US)
(72) Inventor: Shlaferman, Alexander, Brooklyn, NY 11235 (US)
(74) Representative: Hernandez, Yorck

(57) **Abstract**

A device configured for delivery of an aerosol spray payload is disclosed herein. The spray device may be only the size of an ordinary pen, marker, or other writing device. A smaller spray device may be more easily manipulated by the human hand, and may take up a much smaller volume than a typical can of aerosol. Furthermore, the aerosol spray device may be disguised as a pen or marker, so that a bystander may be unable to tell the difference between the spray device and a writing device without close inspection or without removing the cap. The cap and clip may also aid in storage and securing the spray device within a pocket, to a keychain or lanyard, or to another available surface or securing point. The cap may prevent accidental discharge of the spray payload by covering the device's actuator.

## Description

### TECHNICAL FIELD

Aspects of the disclosure relate to a device for delivering an aerosol payload in response to a user's manipulation of, *e.g*., a valve-mounted actuator. The device may be disguised so that it does not appear to be an aerosol device and may be dimensioned to support the disguise and to make manipulation and storage by a user easier.

### BACKGROUND

Aerosol spray devices are used every day to deliver a payload substance quickly and evenly across a target surface. Aerosols are often used for personal and cosmetic reasons, including, for example, hair spray or spray deodorant. Existing aerosol spray devices are typically quite bulky and difficult to store in a pocket, purse, or otherwise on one's person. There is an unmet need for ever smaller, more discreet, and more convenient aerosol spray devices, especially for personal use.

### BRIEF SUMMARY

This Summary provides an introduction to some general concepts relating to this disclosure in a simplified form that are further described below in the Detailed Description. This Summary is not intended to identify key features or essential features of the disclosure.

A device configured for delivery of an aerosol spray payload is disclosed herein. The spray device may be only the size of an ordinary pen, marker, or other writing device. A smaller spray device may be more easily manipulated by the human hand, and may take up a much smaller volume than a typical can of aerosol, making transport and storage of the spray device easier.

Furthermore, the aerosol spray device may be disguised as a pen or marker, so that its purpose is not known until a cap of the spray device is removed. The device may comprise a cap and clip reminiscent of those used to clip a pen in a pocket, or of a particular brand of marker. A bystander may be unable to tell the difference between the spray device and a writing device without close inspection or without removing the cap.

The cap and clip may also aid in storage and securing the spray device within a pocket, to a keychain or lanyard, or to another available surface or securing point. The cap may further aid in preventing accidental discharge of the spray payload by covering an actuator allowing the spray to be released from the device.

In one aspect, the aerosol spray device may include a cylindrical housing tapering to an opening of a smaller diameter, the opening including a raised lip. The cylindrical housing may have a diameter between 10 and 25 millimeters and a length of 70 to 140 millimeters. The device may also include a concealed actuator coupled to the cylindrical housing and configured to release an aerosol spray stored in the cylindrical housing.

In certain embodiments, the concealed actuator is concealed by a cap having a clip oriented parallel or substantially parallel to a central axis of the cylindrical housing. In some examples, the clip has a flange configured to grip a planar surface between the clip and the cap. In some embodiments, the cap has a length of 50 to 60 millimeters and a diameter sized to form a friction fit with the cylindrical housing.

In certain examples, the cylindrical housing has a diameter of between 13 and 19 millimeters and/or a length of 100 to 110 millimeters. In some embodiments, the section of the housing that tapers to the opening is approximately one eighth or less of the cylindrical housing's length. In certain examples, the aerosol spray comprises a propellant and a hair spray. In some embodiments, the concealed actuator is coupled to the cylindrical housing via a metal cylinder crimped over the raised lip.

In various examples, the concealed actuator is revealed by removing the cap, and the concealed actuator may be re-concealed by placing the cap back on the device. In certain examples, the cap has a paraboloid shape tapering along a length of 50 to 70 millimeters from a diameter equal to the cylindrical housing to a rounded top. In some embodiments, the device is shaped, sized, and configured such that the concealed actuator may be actuated by a finger of a user while other fingers and palm of the user completely encircle the diameter of the cylindrical housing.

In another aspect, an aerosol spray device dimensioned for storage in a container configured to hold an ink-filled writing device, or a device configured and dimensioned to resemble an ink-filled writing device is disclosed. In certain examples, the device includes a cap and a clip configured to grasp two sides of a planar surface (such as a cloth pocket) in order to prevent movement of the device. In certain examples, the clip is oriented parallel to a central axis of the device. In some embodiments, the clip grasps one side of the planar surface via a flange extending from the clip towards the cap. In certain embodiments, the device has a cylindrical body with a diameter of between 10 and 25 millimeters and a length of 70 to 140 millimeters. In various examples, the cylindrical body has a diameter of between 13 and 19 millimeters and a length of 100 to 110 millimeters.

### BRIEF DESCRIPTION OF THE DRAWINGS

A more complete understanding of aspects described herein and the advantages thereof may be acquired by referring to the following description in consideration of the accompanying drawings, in which like reference numbers indicate like features. Example embodiments of the disclosure will now be described by way of example only and with reference to the accompanying drawings, in which:
FIG. 1 illustrates an embodiment of a pen-sized aerosol spray device with a cap, according to one or more aspects described herein.
FIG. 2 illustrates an embodiment of a partially unassembled pen-sized aerosol spray device, according to one or more aspects described herein.
FIG. 3 illustrates an embodiment of an assembled pen-sized aerosol spray device with a cap removed, according to one or more aspects described herein.
FIG. 4 illustrates an embodiment of a mechanism for spraying an aerosol payload in response to a user's engaging the device.

### DETAILED DESCRIPTION

In the following description of the various embodiments, reference is made to the accompanying drawings, which form a part hereof, and in which is shown by way of illustration various embodiments in which aspects described herein may be practiced. It is to be understood that other embodiments may be utilized and structural and functional modifications may be made without departing from the scope of the described aspects and embodiments.

Aspects described herein are capable of other embodiments and of being practiced or being carried out in various ways. Also, it is to be understood that the phraseology and terminology used herein are for the purpose of description and should not be regarded as limiting. Rather, the phrases and terms used herein are to be given their broadest interpretation and meaning. The use of "including" and "comprising" and variations thereof is meant to encompass the items listed thereafter and equivalents thereof as well as additional items and equivalents thereof. The use of the terms "mounted," "connected," "coupled," "positioned," "engaged" and similar terms, is meant to include both direct and indirect mounting, connecting, coupling, positioning and engaging.

Moreover, the figures of this disclosure may represent the scale and/or dimensions according to one or more embodiments, and as such contribute to the teaching of such dimensional scaling. However, those skilled in the art will readily appreciate that the disclosure herein is not limited to the scales, dimensions, proportions, and/or orientations shown in the figures.

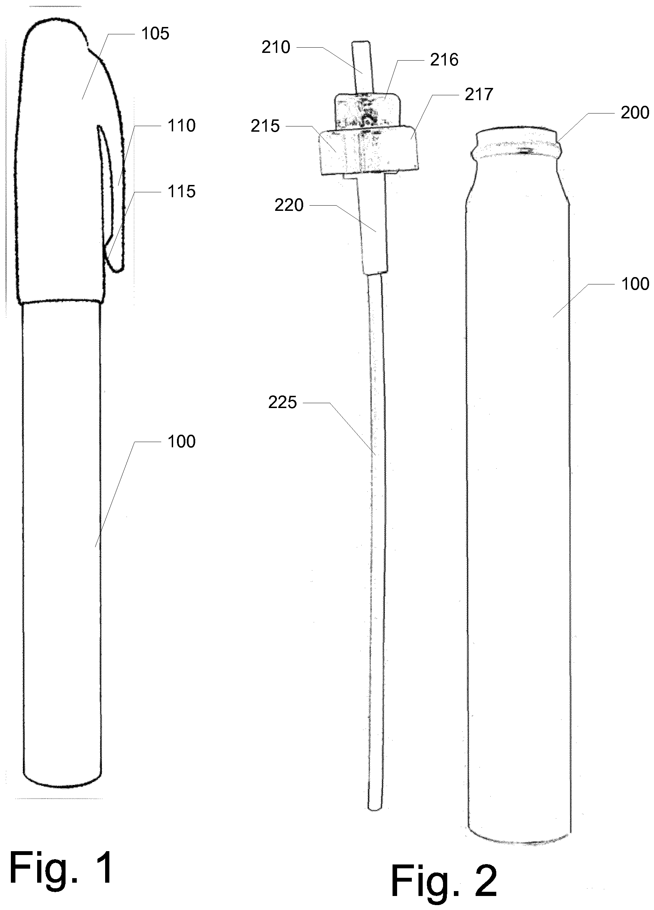

FIG. 1 illustrates an embodiment of a pen-sized aerosol spray device with a cap, according to one or more aspects described herein.

An aerosol spray device may comprise a cylindrical housing 100, which may store a liquid propellant whose violent evaporation causes a powerful spray from the aerosol spray device. The liquid propellant may be mixed with a payload for delivery within the spray. Examples of common payloads may include hair spray, pepper spray, spray paint, cleaning solutions, oils or scented materials, or any other substance desired to be spread evenly over an area and/or dispersed via aerosol. The housing may be made of aluminum, copper, titanium, or any metal, metallic alloys, or plastic strong enough to resist the internal pressure from the propellant.

In some embodiments, the cylindrical housing may have a diameter of 10 to 25 mm, or 13 to 19 mm, or approximately 16 mm. In some examples, the diameter may be 25 mm or less, 20 mm or less, 16 mm or less, or 12 mm or less. The cylindrical housing may have a length of 70 to 140 mm, or 95 to 105 mm, or approximately 100 mm. In other examples, the length may be 140 mm or less, 125 mm or less, or 100 mm or less. In certain embodiments, the dimensions of the diameter and length of the housing (and the other components such as the cap) are selected to resemble or imitate the common features of pen, marker, or highlighter, and therefore may be similar or identical to dimensions of one or more of these devices.

The aerosol spray device, in some embodiments, may comprise a cap 105. The cap may conceal an actuator which is pressed down to release the propellant and payload. The cap may be partially or entirely shaped in a paraboloid fashion, and may run parallel to the housing where it meets the housing (e.g. may have a cylindrical shape to correspond to the section of the housing that forms a friction fit or otherwise engages with the cap), before ending in a rounded tip. In other examples, the cap has a cylindrical section, a tapered section, and a rounded end section.

The cap may be sized to have a diameter approximately equal to that of the cylindrical housing, but slightly larger than the housing diameter, so that the cap will fit snugly (via friction or otherwise) and not be inadvertently removed and the actuator accidentally triggered. In some examples, the housing may comprising a cap lip or ridge, or a channel or indent, configured to interact with a correspondingly shaped engaging portion of the cap (or vice versa). In some examples, the cap and housing may comprising corresponding tab and detent structures. In other examples, the fit will be solely via friction between cylindrically shaped components.

The cap may be pulled or twisted off by a user before using the device, and may be pressed or twisted back on the device when use is complete and the device is to be placed back in storage. In some embodiments, the cap may have a length of 50 to 70 mm, or approximately 55 mm. In other examples, the length is approximately 70 mm or less, or 55 mm or less.

The cap may further have a clip 110 which may resemble clips found on markers, highlighters, or pens. The clip may be adapted to grasp a planar surface when inserted between the cap and clip, and especially the outer edge of a pocket. The clip may further comprise a flange 115 which may, in a resting state, be in contact or almost in contact with the rest of cap 105, and which may grasp the planar surface more firmly than the clip alone when the planar surface is forced between the flange and the rest of the cap. The clip may run parallel to the surface of the cap and the central axis of the housing, such that the device may be clipped in place perpendicular to an edge, surface, or cord grasped by the clip.

The cylindrical housing and cap, taken together, may appear to be a pen or marker, with a cap and clip allowing the device to be worn inside and clipped to a breast pocket or pants pocket, stored inside a pencil bag, makeup bag, or purse, or clipped to any planar surface, such as a collection of sheets of paper or paper money, or to a cord, wire, string, keychain, or lanyard. The device may be stored in a binder or office supply organizer meant for securing a pen or marker within a loop, ring, or specially sized slot or chamber. The device may be shaped in order to emulate a particular marker design in order to appear to a bystander to actually be a genuine marker. In some examples, a bystander may be unable to tell the difference between the spray device and a writing device without close inspection or without removing the cap.

FIG. 2 illustrates an embodiment of a partially unassembled pen-sized aerosol spray device, according to one or more aspects described herein.

In some embodiments, cylindrical housing 100 may taper at the top, such that the top 15%, or 10% of the housing length, or less than 10% tapers to an opening smaller than the diameter of the housing. In other examples, there is no taper and simply a top surface that defines an opening with a smaller diameter so as to accept the other components of the device (as with the tapered embodiments), or a flat surface that extends horizontally to a vertical cylindrical top section that has a smaller diameter, and that in turn has a relatively smaller opening at the top.

The opening may have a diameter of 8 to 15 mm, or approximately 11 mm. In some examples, it may be 15 mm or less, 11 mm or less, or 8 mm or less. A lip 200 may protrude from the tapering surface or a cylindrical surface flowing from the tapered section (having a relatively smaller diameter than the main cylindrical surface). The lip may be adjacent to or otherwise near the opening of the housing. The lip may protrude 1 to 3 mm.

The device may include an aerosol assembly. For example, the device may include a valve assembly comprising valve stem 210, securing cylinder 215, chamber 220, and dip tube 225 may be affixed to the housing to control the output of the propellant and payload. Securing cylinder 215 may be made of copper, plastic, or another malleable or resilient metal or substance, and may comprise an upper portion 216 and a lower portion 217. Upper portion 216 may have a height of approximately 4 mm (or 4 mm or less) and be crimped around valve stem 210 and the top of chamber 220 to secure them in place.

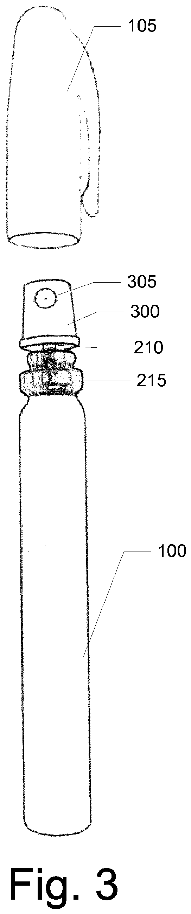

Lower portion 217 may have a height of approximately 5 mm (or 5 mm or less) and be crimped around lip 200 (as shown in FIG. 3) to secure the valve assembly to the housing. These relatively small heights, along with a small height of the valve stem (*e.g.* 6 mm or less, or 4 mm or less) allow the device to more genuinely resemble a writing device. Indeed, by using the combination of components dimensioned in this manner, including the compact aerosol assembly (as described in more detail below) these embodiments allow a very accurate facsimile of a genuine writing device in both size and shape, including the cap of a writing device.

Dip tube 225 may have a length such that, after being secured by the securing cylinder, the dip tube reaches the bottom of the housing, or near the bottom of the housing, to allow payload to pass through the bottom of the dip tube and be sprayed until the housing is fully empty of payload. In examples where the cavity holding the material to be sprayed is less than the entire length of the housing, the tube may be appropriately sized to extend to or near the bottom of the cavity defined by the housing or components and/or surfaces therein.

FIG. 3 illustrates an assembled pen-sized aerosol spray device with a cap removed, according to one or more aspects described herein.

An actuator 300 may be attached to the valve stem 210, so that when the actuator is pressed down, the valve stem is also depressed into chamber 220, creating a channel for propellant and payload to pass up through the dip tube 225, the chamber 220, the valve stem 210, and the actuator itself. The actuator may have an aperture 305 to direct and control the resulting conical spray from the device. The actuator may be about 15 mm or less, 10 mm or less, or 20 mm or less. The actuator may have a dimeter configured to be contained within the cap, and may taper or be otherwise shaped to account for any variances in the shape of the cap (*e.g*. tapering) that allow a closer resemblance to a writing device.

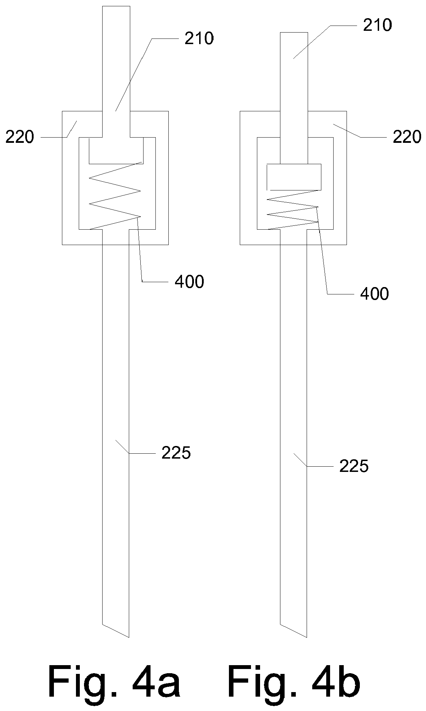

FIGS. 4A-B illustrate a mechanism for spraying an aerosol payload in response to a user's engaging the device.

In a non-spraying state, illustrated by FIG. 4A, a compression element such as spring 400 keeps valve stem 210 pressed against the top of chamber 220, keeping propellant and payload from escaping. The spring may be a coil spring, a leaf spring, or may be in another form. The compression element may comprise a resilient material. When actuator 300 is depressed, in FIG. 4B, valve stem 210 is pressed down, creating an opening for the propellant to force itself and the payload up through the valve stem 225 and out of the aperture of the actuator.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

## Claims

1. An aerosol spray device comprising:
a cylindrical housing, the housing comprising an external diameter between 10 and 25 millimeters, a length of 70 to 140 millimeters and an end section tapering to a reduced diameter, the end section comprising an opening of a second diameter smaller than the external diameter and a raised lip; and
a concealed actuator removably coupled to the cylindrical housing and configured to release an aerosol spray stored within a cavity defined by at least a portion of the cylindrical housing.

2. The device of claim 1, wherein the concealed actuator is concealed by a cap, the cap comprising a cylindrical body defining a cavity configured to accept at least a portion of the cylindrical housing and a clip oriented parallel to a central axis of the cylindrical housing.

3. The device of claim 2, wherein the clip has a flange extending to or near an exterior surface of the cylindrical body of the cap, wherein the clip is configured to grip a planar surface or other objects when positioned between the clip and the cylindrical body of the cap.

4. The device of claim 2, wherein the cap has a length of 50 to 60 millimeters and a diameter sized to form a frictional fit with the cylindrical housing.

5. The device of claim 1, wherein the cylindrical housing has a diameter of between 13 and 19 millimeters.

6. The device of claim 1, wherein the cylindrical housing has a length of 100 to 110 millimeters.

7. The device of claim 1, wherein a length of the end section with the tapering comprises less than one eighth of the cylindrical housing's length.

8. The device of claim 1, wherein the aerosol spray comprises a propellant and a hair spray.

9. The device of claim 1, wherein the concealed actuator is coupled to the cylindrical housing via a metal cylinder crimped over the raised lip.

10. The device of claim 2, wherein the concealed actuator is revealed by removing the cap, and the concealed actuator may be re-concealed by placing the cap back on the device.

11. The device of claim 2, wherein the cap comprises a parabolic shape tapering along a at least a portion of a cap length of 50 to 70 millimeters from a diameter approximately equal to the diameter of the cylindrical housing to a rounded top.

12. The device of claim 1, wherein the device is shaped, sized and configured such that the concealed actuator may be actuated by an index finger of a user while other fingers and palm of the user completely encircle the diameter of the cylindrical housing.

13. An aerosol spray device configured and dimensioned to resemble an ink-filled writing device.

14. The device of claim 13, comprising a cap and a clip configured to grasp two sides of a planar surface inserted between the cap and clip in order to prevent or limit movement of the device.

15. The device of claim 14, wherein the clip is oriented parallel to a central axis of the device.

16. The device of claim 14, wherein the clip grasps one side of the planar surface via a flange extending from the clip towards the cap.

17. The device of claim 14, wherein the planar surface is an outside of a cloth pocket.

18. The device of claim 13, further comprising a cylindrical body with a diameter of between 10 and 25 millimeters and a length of 70 to 140 millimeters.

19. The device of claim 13, further comprising a cylindrical body with a diameter of between 13 and 19 millimeters and a length of 100 to 110 millimeters.

20. The device of claim 13, further comprising a concealed actuator coupled to a cylindrical housing via a metal cylinder crimped over a raised lip of the housing, and wherein the cylindrical housing defines a cavity containing an aerosol spray material, the aerosol spray comprising a propellant and a hair spray.
